# EUROPEAN PATENT APPLICATION

(11) **EP 3 705 879 A1**
(43) Date of publication of application: **09.09.2020**
(21) Application number: 19161038.5
(22) Date of filing: 06.03.2019
(51) Int. Cl.: G01N 27/12, G01N 33/00

(54) **RESISTIVE METAL OXIDE GAS SENSOR**

(71) Applicant: Sensirion AG, 8712 Stäfa (CH)
(72) Inventor: BARTSCH, Sebastian, 8712 Stäfa (CH); EGLI, Daniel, 8712 Stäfa (CH)
(74) Representative: Toleti, Martin

(57) **Abstract**

A resistive metal oxide gas sensor comprises a support structure and a porous sensing layer (1) arranged on the support structure or partly housed therein. Electrodes (2) are in electrical communication with the porous sensing layer (1), and a heater (3) is in thermal communication with the porous sensing layer (1). The heater (3) can be operated to heat the porous sensing layer (1) to a target temperature for allowing a determination of the presence or the concentration of a target gas, i.e., ozone, based on a sensing signal supplied via the electrodes (2). The porous sensing layer (1) comprises a network of interconnected monocrystalline metal oxide nanoparticles (14) and a gas-selective coating (12) of the network. A thickness (t1) of the porous sensing layer (1) is at most 10 µm. The coating (12) comprises one or more of silicon oxide and silicon nitride, and is of a thickness (t12) of at most 10 nm.

## Description

### Technical Field

The invention relates to the field of resistive metal oxide gas sensors, to a method for operating a resistive metal oxide gas sensor, and to a method for manufacturing a resistive metal oxide gas sensor.

### Background Art

Gas sensors include a sensing (or active) layer or film sensitive to the presence or concentration of one or more gases. One class of gas sensors involves chemiresistors, i.e., materials for which the electrical resistance changes in response to changes in their direct chemical environment.

Chemiresistors are sometimes defined as relying on direct chemical interactions between the sensing material and the gaseous analyte/s. More general definitions of chemiresistors, however, include materials for which the electrical resistance changes in response to any type of interactions (chemical, hydrogen bonds, van der Waals, etc.) in their direct environment. In each case, the sensing material may contain a metal oxide material.

In metal oxide sensors, gaseous analytes interact with the heated metal oxide layers. As a result of the interaction, the conductivity of the sensitive layer or film may change, and the change may be measured. Such gas sensors are also referred to as "high temperature chemiresistors" for the reason that a chemical property of the analyte is converted into an electrical resistance at high temperatures of the sensitive film.

Such sensitive layer or film requires a heater to heat the sensing material. It thus can be integrated onto a semiconductor substrate, in which case the heater is advantageously arranged on a bridge or a cantilever over an opening in the semiconductor substrate, thereby reducing the thermal loss as compared to devices where the heater is arranged over the bulk of the substrate material. Arranging the heater on a membrane has several advantages, such as reducing power consumption and reducing the time required for switching on the device.

WO 2018/053655 A1 refers to a resistive metal oxide gas sensor with a support structure and a patch of sensing material arranged on the support structure or partly housed therein. The patch comprises a metal oxide material. Electrodes are in electrical communication with the patch. The sensor further comprises a heater, in thermal communication with the patch, and a selective gas-permeable filter. The selective gas-permeable filter comprises a fluoropolymer.

### Disclosure of the Invention

The problem to be solved by the present invention is to provide a metal oxide gas sensor that is sensitive to ozone and shows an improved selectivity and / or improves long-term stability.

This problem is solved by a resistive metal oxide gas sensor according to the features of claim 1. This resistive metal oxide gas sensor is capable of sensing ozone as target gas and / or decomposition products such as atomic oxygen. Specifically, the resistive metal oxide sensor is capable of detecting a presence or a concentration of the target gas in an environment of the resistive metal oxide gas sensor. In embodiments, the resistive metal gas sensor is designed to sense hydrogen molecules, in addition to or in variants to sensing ozone.

The gas sensor comprises a support structure for supporting a sensitive element, the latter also being referred to as sensing layer or sensitive film. The sensing layer is a porous layer and comprises monocrystalline metal oxide nanoparticles. Other than the monocrystalline metal oxide nanoparticles, the porous layer also comprises void space leading to the porous characteristic of the sensing layer.

A monocrystalline metal oxide nanoparticle, in the following abbreviated as monocrystalline MOX NP, is considered a small particle that contributes to the porous sensing layer in combination with a multitude of other monocrystalline MOX NPs, preferably of the same metal oxide material. Preferably, the average size of the monocrystalline MOX NPs is at most 100 nm, preferably at most 50 nm, more preferably within a range from 5 nm to 19 nm, and even more preferably within a range from 10 nm to 15 nm. The relevant size preferably is a diameter of the monocrystalline MOX NP. In case of monocrystalline MOX NPs without a uniform diameter, a longest extension of the monocrystalline MOX NP is considered to be the relevant size. Thus, the average size of the monocrystalline MOX NPs can be regarded as the average of the largest characteristic dimensions of such particles. Preferably, the size of a monocrystalline MOX NP / the monocrystalline MOX NPs is measured by X-ray diffraction (XRD), SEM and/or TEM. Preferably, the average size of the monocrystalline MOX NPs is determined by the arithmetic mean. However, in variants, the average size may also be represented by the median in the statistical distribution of the sizes of the monocrystalline MOX NPs, for example. Relying on such NP sizes increases the surface to volume ratio, which, in turn, increases the sensitivity of the sensor to the target gas/es.

In one embodiment, the metal oxide material used for the nanoparticles consists of one of tin oxide, zinc oxide, titanium oxide, tungsten oxide, indium oxide and gallium oxide. In another embodiment, the metal oxide material used for the nanoparticles comprises one or more of tin oxide, zinc oxide, titanium oxide, tungsten oxide, indium oxide and gallium oxide. Preferably, the monocrystalline MOX NPs additionally comprise a noble metal of at most 10 wt% (percentage by mass), and preferably between 0.3 wt% - 4.0 wt%. All values of percentages by mass mentioned herein correspond to mass ratios expressed in percent and are subject to ± 0.1 wt%. The noble metal in one embodiment is one of Pt, Pd, and Ir. The metal oxide material may in one embodiment be doped by the noble metal. Note, "doping" is to be understood in a broad sense and may notably include an impregnation process. Accordingly, the metal oxide material may for instance be impregnated by the noble metal. In fact, doping shall encompass all variants of adding such a noble metal to the MOX NPs, such as, but not limited to bulk loading, surface loading, and/or cluster doping by using only few atomic species for doping. Noble metal particles at the surface of the monocrystalline MOX NPs may serve as nuclei for depositing a coating material to be introduced later.

The average size of the monocrystalline MOX NPs is relevant inasmuch as it defines a maximal surface of the sensitive material the gaseous analyte/s can interact with. In the present context, the average size of the monocrystalline MOX NPs will ensure sufficient surfaces for chemical and/or other interactions between the target gas and the sensitive material.

The monocrystalline MOX NPs are arranged on the support structure such that they are interconnected. I.e., adjacent NPs are in mechanical contact, although it is not necessary that each monocrystalline MOX NP is connected to each of its adjacent monocrystalline MOX NPs. However, for the overall functionality of the gas sensor, an electrically conducting path is required from one electrode to the other for measuring the conductivity of the porous sensing layer in between. Hence, and given that the formation of interconnects between monocrystalline MOX NPs strongly depends on the manufacturing / deposition process, a sufficient condition may be for this process to ensure that a monocrystalline MOX NP be linked to at least one adjacent monocrystalline MOX NP with a given, minimal probability. Such interconnects do not necessarily require substantial chemical bondage between adjacent monocrystalline MOX NPs. The interconnecting property may in fact arise from electrical interconnects between adjacent monocrystalline MOX NPs, where such electrical interconnects are ensured by direct mechanical contact rather than linkage. Such interconnects between adjacent monocrystalline MOX NPs are generated after depositing the monocrystalline MOX NPs onto the support structure, i.e., in an annealing or sintering step. For the present purposes, an annealing process is considered to be carried out at temperatures below 700 °C, while a sintering process is considered to be carried out at temperatures above 700 °C. Under impact of heat, the monocrystalline MOX NPs permanently arrange in contact with each other such that sufficient electrical interconnects can be obtained. After annealing or sintering, the monocrystalline MOX NPs build a network of interconnected monocrystalline MOX NPs. Only this network is then coated as explained below, such that the coating does not affect the electrical connection between adjacent monocrystalline MOX NPs. The monocrystalline property of the interconnected MOX NPs enhances the electrical properties of the semiconducting metal oxide material and, in particular, supports the electrical conductivity of the network (as a whole).

A coating is provided for the network of interconnected monocrystalline MOX NPs. Both the network of interconnected monocrystalline NPs and the coating of the network contribute to the porous sensing layer. The coating is used as a gas-selective means (i.e., a filter layer). The coating may inhibit or reduce the interaction of one or more gaseous analytes other than the target gas/es with the metal oxide material. The target gas/es is ozone, and preferably hydrogen (i.e., H2 molecules), while the other gaseous analytes to be excluded or minimized from a reaction with the metal oxide material may include one or more of, and preferably all of, volatile organic compounds (VOCs), hydrocarbons, carbon monoxide, nitrogen dioxide, methane, ammonia, and hydrogen sulphide. In embodiments, the coating is designed so as for these gaseous analytes to be substantially blocked by the coating from reaching the sensitive material.

The coating comprises one or more of silicon oxide and silicon nitride. In one preferred embodiment, the coating is made from silicon oxide only, while in a different embodiment, the coating is made from silicon nitride only. Silicon oxide is understood as any compound of silicon and oxygen, i.e., SiOx, and in particular includes silicon dioxide SiO2, i.e., silica. Silicon nitride is understood as any compound of silicon and nitrogen, i.e., SiNx, and in particular includes Si3N4.

Preferably, the coating is applied to all surfaces of the network accessible by gaseous precursors of the coating material, i.e., is not only applied to a top surface of the porous sensing layer, but also to surfaces inside the porous sensing layer, it being noted that the gaseous precursor/s of the coating material may diffuse into voids of the porous sensing layer, so as for the coating to eventually cover all surfaces accessible therein. Accordingly, it is preferred that the coating applies to top surfaces of the network facing the environment prior to coating as well as to surfaces of the network buried in the porous sensing layer but accessible through the voids of the porous sensing layer.

Since in view of the manufacturing process other surfaces of the network of monocrystalline MOX NPs may yet be covered by other materials, and in particular by gas-tight materials, it is preferred that surfaces in direct contact with the support structure remain uncovered by the coating. So do surfaces of the network in direct contact with the electrodes, and possibly the heater in case of a direct contact there between. And surfaces of the network facing voids in the porous sensing layer inaccessible for the gaseous precursors may remain uncovered by the coating too, given that the gaseous precursors of the coating do not reach such "closed" voids in the porous sensing layer. These embodiments provide for an overall sealed metal oxide material, either by means of the coating, or by other gas-tight materials such that no bypass is offered to the metal oxide material other than through the coating. Given that the coating is gas-selective, it provides only for the target gas/es to reach the metal oxide material while other gaseous analytes are blocked from access to metal oxide material and therefore are prevented from impacting the measurement result.

In a preferred embodiment, the coating is uniform in its thickness, subject to a tolerance of ± 50%, and preferably to a tolerance of ± 20%. A rather uniform thickness of the coating across all coated surfaces of the network supports a common reaction time for all target molecules irrespective at which location they meet the coating (i.e., this narrows down the reaction time range), and also provides a common gas-selectiveness across all coated surfaces. Such tolerances are determined by the actual manufacturing process selected. In some embodiments, however, a non-uniform thickness may be accepted in case, for example, the MOX NPs are impregnated with noble material particles serving as nuclei for growing the coating on the metal oxide material. In particular, in the surrounding of these nuclei, the coating may be outside the above tolerances. However, other than at these nuclei locations the coating may still be preferred to show a rather uniform thickness, subject to tolerances as given above. Absent such nuclei, the coating may be preferred to be a conformal coating, i.e., uniform in thickness and following the structure / shape of the underlying network of interconnected MOX NPs. Preferably, the coating is continuous, i.e., free of holes or gaps other than the pores for the target gas. Continuous in this context shall mean that at least 90% of the entire surface of the network that is accessible to gaseous precursors of the coating and that is not covered by any of the support structure, the electrodes or the heater, is covered by the coating. Continuous shall preferably encompass that also surface transitions between interconnected adjacent individual monocrystalline MOX NPs are covered by the coating.

The effect of the coating may, according to one interpretation, rely on filtering. The coating may accordingly act as a molecular sieve that selects molecules or atoms dependent on their size. According to another interpretation, the coating can be regarded as a means for deactivating the most active sites on the surface of the metal oxide material. Active sites allow gaseous analytes to be detected by triggering interaction between the latter and the sensing layer to generate a sensing signal. Ozone, and / or in particular its decomposition products, and also hydrogen, have a small molecular / atomic size compared to other gaseous analytes, and, at the same time, show a high chemical activity, including reactivity. These properties of the target gas/es allow them to pass the coating irrespective of any of the above interpretations. According to the first interpretation, ozone, and / or in particular its decomposition products, is/are small enough to pass the pores in the coating material while other gaseous molecules are not. According to the second interpretation, ozone is sufficiently reactive to still react with the metal oxide material although the coating serves as passivation. In the latter case, ozone additionally shows a sufficient penetration depth into the porous sensing layer for significantly affecting the conductivity of the metal oxide material.

In any case, the coating was found to have a gas selective property, and irrespective of the above interpretations, the coating selectively grants access to the coated metal oxide material subject to size and / or reactivity. In any case, molecules of a sufficient small size and high reactivity, and in particular ozone, and / or its decomposition products, can reach the sensitive material through the coating.

A thickness of the coating is at most 10 nm. Preferably, the thickness of the coating is less than 5 nm, and more preferably less than 5 nm and more than 1 nm. Note, the values expressed herein in nanometres are assumed to be subject to an accuracy of ± x nm, where 0 < x < 1 nm. On the one hand, such a thickness allows a diffusion of ozone, oxygen, and / or other decomposition products into the metal oxide material. On the other hand, the coating may have a sufficient thickness to bar other molecules from penetrating into the metal oxide material.

As another measure of the gas sensor, a thickness of the porous sensing layer is at most 10 µm. In another variant, a thickness of the porous sensing layer is at most 5 µm, and preferably is at most 1 µm. Preferably, a minimum thickness of the porous sensing layer is 100 nm, consistently with preferred dimensions for the NPs.

Note, the values expressed herein in micrometres are generally assumed to be subject to an accuracy of ± 0.1 µm, except where the same or corresponding values are otherwise expressed in nanometres. The thickness of the porous sensing layer preferably is an extension of the layer in z-direction in case a surface of the support structure (which surface supports the porous sensing layer) extends in x- and y-direction. The thickness of the porous sensing layer preferably is an average thickness in case the thickness varies across the porous sensing layer. The average thickness preferably is an arithmetic mean of varying thicknesses of the porous sensing layer, if any. The thickness of the porous sensing layer includes an overall thickness including the network of interconnected monocrystalline MOX NPs and the coating of the network given that both the monocrystalline MOX NPs and the coating contribute to the layer.

The thickness of the porous sensing layer does, in combination with other parameters listed in claim 1, critically impact the sensitivity of the gas sensor to ozone, as well as its selectivity for ozone versus many other gaseous analytes. In a porous sensing layer thicker than suggested above, ozone molecules would only react in areas of the metal oxide material that are close to a top of the porous sensing layer but not in the bulk of the porous sensing layer. Hence, only a small fraction of the total amount of metal oxide material would experience an interaction with the target gas. And this would not be beneficial in view of the resulting low sensitivity of the gas senor. This is all the more true if the electrodes are arranged at a bottom of the porous sensing layer while the target gas is assumed to meet the porous sensing layer at its top opposite the bottom (the top and bottom sides extending, each, perpendicular to the z-direction). In such cases, the change in conductivity of the metal oxide material would originate far from the electrodes and may thus not sufficiently contribute to the sensing signal. Accordingly, it is desired to bring the electrodes closer to the area where reactions take place. This is achieved by dimensioning the porous sensing layer thickness as thin as suggested above, in combination with other parameters as listed in claim 1, since the thicker the porous sensing layer, the less the fraction of the total metal oxide material that reacts. By means of the suggested thin dimensioning of the porous sensing layer, ozone molecules may enter the porous sensing layer with a significant penetration depth in relation to the overall thickness of the porous sensing layer. Accordingly, the electrodes can sense a change in conductivity or resistance not only in a small fraction of the porous sensing layer but in a larger fraction, such that the sensitivity of the sensor is significantly increased.

In a preferred embodiment, the porous layer contains a void fraction between 30% and 60% (±10%), which void fraction denotes the porosity of the layer. The void fraction may for example be between 30% and 40%. Preferably, the value of porosity refers to the coated monocrystalline MOX NPs. In view of the reactive property of ozone, a rather large porosity such as noted above promotes a sufficient penetration depth of the target gas molecules into the layer, and accordingly enables a penetration depth closer to the electrodes, which is beneficial for increasing sensitivity.

A coating thickness as laid out above does not compromise the porosity of the sensing layer. Such a thin coating does not clog the voids and does not lead to the same effect as if the porosity were chosen too small from the beginning, or if the porous sensing layer were selected too thick from the beginning. Preferred thicknesses for the coating and porosity as set forth above may be leveraged to impact the diffusion of the target gas into lower regions of the porous sensing layer.

In addition, the rather large porosity may also facilitate the manufacturing of the coating. A lower porosity would adversely affect the depth of penetration while an even larger porosity would in turn reduce the active surface of the coated monocrystalline MOX NPs and again result in a lower sensitivity.

The porous sensing layer is arranged on the support structure or partly housed therein. Electrodes are provided in electrical communication with the porous sensing layer, in particular for measuring e.g., a current through the porous sensing layer by applying a voltage, and for supplying e.g., the measured current as a sensing signal indicative of the electrical conductivity or the electrical resistance of the porous sensing layer. The sensing signal is not limited to an analogue signal over time, but shall also include a processed analogue signal, e.g., one or more values sampled by an A/D converter. Preferably the sensing signal represents a measure for the target gas to be detected.

In one embodiment, the electrodes are arranged on or in the support structure and are in electrical contact with the porous sensing layer, and in particular with the metal oxide material of the porous sensing layer. This may in particular be achieved if the monocrystalline MOX NPs are deposited on the support structure including the electrodes prior to coating.

In addition, a heater is provided in thermal communication with the porous sensing layer. The heater is arranged and designed to heat the metal oxide material in order to enable a measurement of the target gas. In one embodiment, the one or more heaters are arranged on or in the support structure. However, there is no need for a direct contact between the heater and the porous sensing layer as long as sufficiently heat is transferred to the porous sensing layer.

For operating the gas sensor in a way that enables the detection of ozone, a controller may for instance be provided (e.g., integrated in the sensor, or not), which is configured to control and / or operate the heater to heat the porous sensing layer to a target temperature, as in embodiments. That the porous sensing layer reaches the target temperature makes it possible to receive a meaningful sensing signal from the electrodes, where this signal is representative of the concentration of the target gas to be detected or, at least, indicative of the presence of molecules of this gas. In one embodiment, the target temperature not only is reached for an instant but may be maintained for a period in time. The sensing signal supplied via the electrodes may in one embodiment be accepted as measurement value as soon as the porous sensing layer reaches the target temperature. In a different embodiment, the sensing signal is accepted as measurement value - i.e., is processed for the determination of the target gas concentration - at a defined period in time after the target temperature has been reached and is maintained. Or, in another embodiment, the sensing signal is accepted as measurement value at a defined period in time after the target temperature has been reached irrespective if the heater continues heating the porous sensing layer after the target temperature has been reached.

The target temperature of the porous sensing layer during a sensing operation is in a temperature range between 400 °C and 600 °C, and preferably between 450 °C and 550 °C.

Again, the dimensioning of the temperature range for the target temperature is crucial in particular for achieving a sufficient sensitivity to the target gas ozone. A temperature outside the above range, and in particular higher than the suggested upper bound, would significantly lower the sensitivity. At such high temperatures, the ozone may on the one hand diffuse into the porous sensing layer at high speed and, however, may react very swiftly such that the reaction may exclusively take place in the top region of the porous sensing layer. Such high temperatures do not allow ozone to reach a sufficient penetration depth in the porous sensing layer to make up a sensing signal of sufficient strength. On the other hand, temperatures lower than the lower bound of the above temperature range significantly impact a reaction time of the gas sensor. Such low temperatures would slow down the diffusion of the ozone through the coating. The interaction with the sensing material would only be achieved long after having exposed the gas sensor to the target gas.

Accordingly, the ranges for the porous sensing layer thickness, the porous sensing layer porosity, the coating thickness, and preferably the operating temperature determine characteristics of the sensor. Such parameters can be refined to optimize the ability of the gas sensor to sense ozone and/ or hydrogen, as in embodiments contemplated herein. An appropriate combination of such parameters need be achieved, which takes into account critical properties of the target gas molecule/s, including the size of the target gas molecule and their reactivity. Ozone is considered to be very reactive and relatively small, while hydrogen is considered to be very small and relatively reactive. A specific design as laid out above (in respect of the coating, its material, the network of the MOX NPs, and their material) determines the kind of molecules (in size and reactivity, preferably in combination) that are allowed to diffuse through the coating to the MOX NPs and at the same time diffuse with a sufficient penetration depth into the porous sensing layer in order to generate a meaningful, i.e., discernible signal. This design is preferably such as to only allow detection of ozone and/or hydrogen molecules (or decomposition products thereof), subject to water molecules that may also happen to be systematically detected by the process.

In embodiments, two diffusion effects are likely to cause the target gas/es to generate a meaningful sensing signal. First, on the microscopic scale of individual MOX NPs, the diffusion of the target molecules is preferably required through the coating in order to interact with the MOX NPs. Second, on the macroscopic scale of the entire porous sensing layer, a sufficient amount of target gas molecules are preferably required to diffuse into deeper portions of the porous sensing layer towards the electrodes in order to generate interactions with MOX NPs in a region that affects the resistance measured by the electrodes. Note, the effects and properties of the diffusion and reactivity strongly depend on the temperature of the gases involved and consequently on the temperature of the porous sensing layer, whence the need for controlling the temperature range, as discussed above.

In one embodiment, the controller is embodied as an electronic circuit or processing unit connected to the heater, and is programmed, designed, adapted or configured to operate the heater for heating the porous sensing layer to the target temperature in the specified temperature range, and in another embodiment is additionally embodied to prevent the heater to heat the porous sensing layer beyond the specified target temperature range. Preferably, the controller is also configured to receive the sensing signal supplied via the electrodes and, for example, to A/D convert the sensing signal into values of a discrete sensing signal, and / or further pre-process and evaluate the sensing signal. In one embodiment, the support structure may contain a semiconductor substrate, and the controller may be integrated as electronic circuit into the semiconductor substrate which substrate at the same time supports the porous sensing layer. In a different embodiment, the controller may be embodied in a chip separate from the support structure, such as in an ASIC, and may only be electrically connected to the support structure. Support structure and ASIC then contribute to the gas sensor.

In more sophisticated embodiments, the gas sensor further comprises a temperature sensor arranged in the resistive metal oxide gas sensor for estimating a temperature of the porous sensing layer. The temperature sensor may form part of the heater. In this embodiment, the controller is preferably connected to the heater and to the temperature sensor. The controller may form, together with the heater and the temperature sensor, a feedback loop, to make it possible to maintain a temperature of the porous sensing layer at a substantially constant value of the target temperature.

According to another embodiment, a "multipixel" resistive metal oxide gas sensor is provided, to concomitantly sense several types of gas molecules. Namely, such a sensor comprises one or more support structures and a set of films of sensing material electrically disconnected from each other, wherein preferably one of the films is a porous sensing layer as discussed in the previous embodiments. Each of the films comprises a metal oxide material and is arranged on or is partly housed in the one or more support structures. In addition, a set of electrodes is provided, whereby each of the films is in electrical communication with a subset of the electrodes. One or more heaters are in thermal communication with the films. Finally, this gas sensor comprises one or more gas selective coatings preferably of different selectivity. The films preferably differ in terms of dimensions and/or compositions of their respective coatings and/or the respective metal oxide materials, or in the configuration of the electrodes, from one film to the other, so as to be able to sense different types of gases.

The present invention may for instance be embodied as an electronic device, in particular a home automation device, a consumer electronics device, a mobile phone, a tablet computer or a watch, comprising any resistive metal oxide gas sensor such as discussed above.

According to another aspect, the invention is embodied as method of operating a resistive metal oxide gas sensor according to any of the above embodiments. Basically, such a method revolves around taking a measurement by heating the porous sensitive layer to the target temperature, and by determining a presence or a concentration of the target gas based on the sensing signal received from the electrodes, preferably while heating the porous sensing layer. The sensing signal may include or be represented by one or more values indicative of the electrical conductivity of the porous sensing layer.

In a preferred embodiment, prior to taking a measurement as laid out above, or even prior to taking any measurement, i.e., prior to operating the gas sensor in a measurement environment, the gas sensor is calibrated by placing the gas sensor in a gas mixture including hydrogen. The sensor is calibrated by taking into account a hydrogen background concentration as expected in the measurement environment. Given that a gas sensor of one or more of the embodiments as laid out above may also be sensitive to H2 next to ozone, in particular in view of a similar size of the respective molecules and a similar reactivity, both analytes, i.e., ozone and hydrogen, if present in the environment of the gas sensor may interact with the sensing material of the layer. However, given a background concentration of hydrogen is known for typical outdoor environments, a share of the hydrogen in the sensing signal can be determined upfront during a calibration procedure such that the calibrated sensing signal represents a sensing signal free of the hydrogen contribution. Accordingly, for an anticipated measurement environment including a hydrogen background concentration, the calibration may be performed, after having placed the sensor in a gas mixture comprising hydrogen. The same calibration method may be applied in case the measuring environment is expected to contain an oxygen background concentration. Both, hydrogen and oxygen background concentrations may be found in ambient air, and a calibration to both background concentrations is preferred in case the anticipated measurement environment for the gas sensor is ambient air. In case the anticipated environment for the gas sensor is an environment free of hydrogen and / or oxygen background concentration, the respective share is set to zero in the manufacturing procedure, and / or the calibration procedure.

According to another aspect of the present invention, a method is provided for manufacturing a resistive metal oxide gas sensor sensitive to ozone. A support structure is manufactured including electrodes and a heater. Monocrystalline MOX NPs are deposited on the support structure, wherein the average size of the monocrystalline MOX NPs is preferably at most 100 nm. After having deposited the monocrystalline MOX NPs on the support structure, e.g., by one of FSP, screen printing of ink jet printing, the monocrystalline MOX NPs are annealed or sintered thereby forming a network of interconnected monocrystalline MOX NPs. After the annealing step, a gas-selective coating of a thickness of at most 10 nm is applied on the network of interconnected monocrystalline MOX NPs. The coating comprises one or more of silicon oxide and silicon nitride. Both the monocrystalline MOX NPs and the coating contribute to a porous sensing layer of a thickness of at most 10 µm. The porous sensing layer is arranged on the support structure or partly housed therein such that the electrodes are in electrical communication with the layer and such that the heater is in thermal communication with the layer.

Preferably, the coating is applied by means of a chemical vapour deposition (CVD) process at a coating temperature in a coating temperature range between 400 °C and 700 °C, and specifically in a coating temperature range between 500 °C and 600 °C. The heating for the CVD process preferably is performed by the heater of the gas sensor. Accordingly, the heater is operated to achieve a coating temperature in the above coating temperature ranges.

Preferably, for the CVD process a gaseous oxygen species and/or a gaseous nitrogen species is/are applied as gaseous precursor/s as well as a gaseous silicon species. The CVD process preferably is applied at ambient pressure, in particular at a pressure of 1 atm. Preferably, the gaseous oxygen species comprises one or more of 02 and 03 and/or the gaseous nitrogen species comprises ammonia, and preferably is/are comprised by zero air. Preferably, the gaseous silicon species is one or more of silane, dichlorosilane, tetraethylorthosilicate, or TEOS, hexamethyldisiloxane, or HMDSO, hexamethylcyclotrisiloxan, or D3, octamethylcyclotetrasiloxan, or D4, and decamethylcyclopentasiloxane, or D5.

The coating temperatures as well as the coating periods as suggested are outside the coating temperatures and periods of conventional CVD processes. However, the subject coating temperature ranges are found to be preferred for the diffusion of the source gases into the voids between the deposited monocrystalline MOX NPs to build the coating even there and not only on the top surface of the deposited monocrystalline MOX NP assembly. Lower coating temperatures, on the other hand, would significantly increase the coating periods. Higher coating temperatures on the other hand may chemically reduce the metal oxide material of the monocrystalline MOX NPs.

Other advantageous embodiments are listed in the dependent claims as well as in the description below.

### Brief Description of the Drawings

Devices, apparatuses and methods embodying the present invention will now be described, by way of non-limiting examples, and in reference to the accompanying drawings, wherein:
FIG. 1 is a cross-sectional view of a gas sensor, according to an embodiment of the present invention;
FIG. 2 is a cross-sectional zoomed-in view of region 1z of FIG. 1, according to an embodiment of the present invention;
FIG. 3 is a cross-sectional zoomed-in view of region 1z of FIG. 1, according to another embodiment of the present invention;
FIG. 4 is a cross-sectional view of a gas sensor, according to another embodiment of the present invention;
FIG. 5 is a top view of the gas sensor of FIG. 4;
FIG. 6 is a top view of a gas sensor, according to a further embodiment of the present invention;
FIGs. 7 to 9 show plot representing measured resistances of a gas sensor according to an embodiment of the present invention after exposure to different types of gas molecules;
FIG. 10 shows a plot representing measured resistances of a gas sensor according to an embodiment of the present invention in a long term measurement; and
FIG. 11 is a flowchart illustrating high-level steps of a method of operating a gas sensor device, according to embodiments.

The accompanying drawings show simplified representations of devices or parts thereof, as involved in embodiments. Technical features depicted in the drawings are not necessarily to scale. Similar or functionally similar elements in the figures have been allocated the same numeral references, unless otherwise indicated.

### Modes for Carrying Out the Invention

Embodiments of the metal oxide (MOX) gas sensors according to the present invention are attractive because of their small footprint, fast response, and high sensitivity to the desired target gas/es. In addition, previous drawbacks of conventional MOX gas sensors are overcome by the present embodiments: The selectivity achieved for the target gas/es is raised, and long-term drift due to poisoning by silicone compounds is significantly reduced such that no or almost no deactivation of the catalytic activity occurs over time.

The gas sensor according to embodiments comprises a porous sensing layer of a semiconducting metal oxide, such as SnO2, including monocrystalline metal oxide nanoparticles, in following monocrystalline MOX NPs, with an average size typically in the tens of nm. The monocrystalline MOX NPs are assembled in network of interconnected monocrystalline MOX NPs, such that electrical conductivity is provided between the electrodes through the network of interconnected monocrystalline MOX NPs. The interconnectivity may be achieve in an annealing or sintering step applied after having deposited the monocrystalline MOX NPs on the support structure.

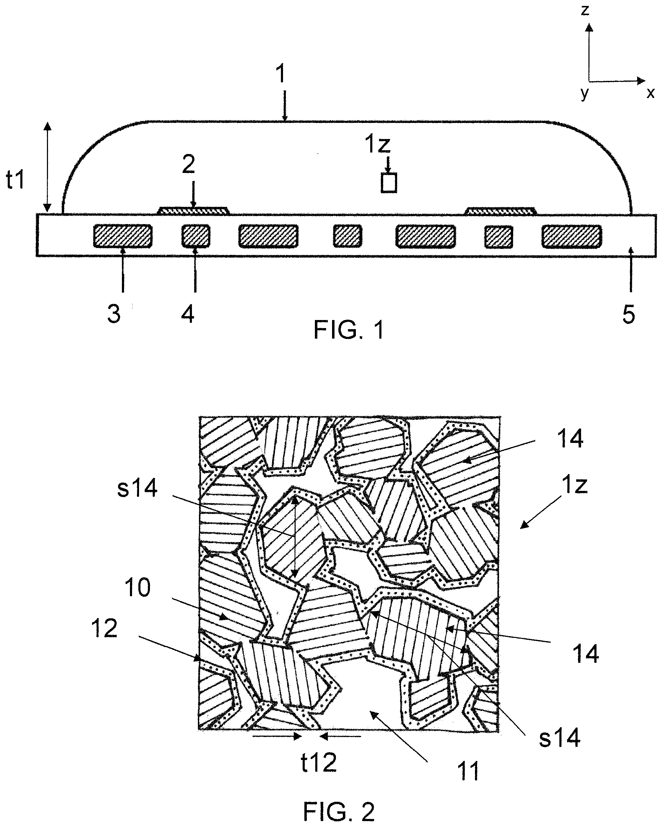

FIG. 1 illustrates a cross-sectional view of a gas sensor according to an embodiment of the present invention. The gas sensor comprises a support structure which presently is embodied as a substrate 5, and more preferably as a silicon substrate. The substrate 5 has a planar extension in x and y direction, and a height in z-direction.

A couple of elements are integrated in the substrate 5, which elements comprise one or more resistive heaters 3, and one or more temperature sensors 4. The various elements referencing a heating structure in one embodiment can represent a single heater 3 with multiple arms electrically interconnected, and hence controllable by a single controlling signal, or, in a different embodiment can represent multiple heaters individually controllable. Preferably, the heater 3 is made from tungsten. The temperature sensor 4 is preferably also made from tungsten and is provided for monitoring the temperature of the substrate 5 which may at the same time represent the temperature of a porous sensing layer 1 deposited on the substrate.

The porous sensing layer 1 is to be heated to a target temperature within a target temperature range. Accordingly, a controller (not shown, e.g., provided as part of the sensor or on a separate chip) controls the heater 3 to heat the porous sensing layer 1 to the target temperature while the temperature sensor 4 monitors the heating efforts. Subject to the temperature sensed by the temperature sensor 4, the heater is controlled e.g., to continue heating, or, e.g., to stop heating, in a feedback loop in which the target temperature is the set value to be compared to the actual value supplied by the temperature sensor 4.

Electrodes 2 are arranged on top of the substrate 5. Preferably, the electrodes 2 are made from platinum. Electrodes 2, heater 3, and temperature sensor 4, if any, may be manufactured within a conventional CMOS process for processing a CMOS layer stack on top of a Si wafer. In the present example, the heater 3 and the temperature sensor 4 may be manufactured from one or more conducting layers within a CMOS layer stack of conducting and insulating layers. The electrodes may also be made from one of the conducting layers of the layer stack, or may be made from a conducting material, such as Pt, applied on top of the CMOS layer stack. The electrodes 2, the heater 3 and / or the temperature sensor 4 may be shaped by lithography steps, for example.

The porous sensing layer 1 is arranged on top of the support 5 and covers at least part of the electrodes 2. The porous sensing layer 1 shows an extension in x- and y-direction and has a thickness in z-direction referred to by t1. The thickness t1 of the porous sensing layer 1 is less than 10 µm, and in the present example e.g., is 5 µm.

The controller is configured to operate the heater 3, and to specifically heat the layer 1 to a target temperature in a temperature range between 450 °C and 550 °C. In response to such heating, the electrodes supply an electrical sensing signal to the controller for further processing and / or evaluation. The sensing signal is representative of the electrical conductivity or the resistance of the porous sensing layer 1 in between the electrodes 2. In view of the heating of the porous sensing layer 1, ozone as a target gas may reach metal oxide material contributing to the porous sensing layer 1, interact there and lead to a change in the electrical conductivity thereof.

Reference sign 1z denotes an arbitrary region in the porous sensing layer 1, a zoomed view of which arbitrary region is illustrated in Figure 2 in cross-section. As can be derived from Figure 2, the sensing layer 1 is a porous layer given that the white spaces in Figure 2 denote voids 11 while the dashed areas denote metal oxide material 10. The metal oxide material 10 preferably is SnO2 in the present example. The dotted areas refer to a coating 12 the metal oxide material 10 is covered with. In the present example, the coating 10 is made from Si02. The metal oxide material 10 has the shape of monocrystalline metal oxide nanoparticles 14, referred to as monocrystalline MOX NPs. The different orientation of the dashes from MOX NP to MOX NP shall illustrate the monocrystalline property of each individual MOX NPs, in order to distinguish from a monocrystalline network which presently is not the case.

Adjacent monocrystalline MOX NPs 14 are electrically connected to each other, i.e., they may contact each other, but are not necessarily mechanically linked to each other. The monocrystalline MOX NPs 14 thus form a network of interconnected monocrystalline MOX NPs 14. A coating 12 coats the network without coating the contacts between adjacent monocrystalline MOX NPs. The coating 12 is a gas-selective coating and presently is selective for ozone.

In the present example, the coating 12 is considered a uniform coating, i.e., a coating of rather uniform thickness including a tolerance of ± 50%, and a conformal coating that follows the contour of the coated material by not changing its thickness within the tolerances. The coating of the present example may also be essentially continuous, i.e., free of holes or gaps other than the pores for the target gas. As can be derived from FIG. 2, all surfaces of the network of interconnected monocrystalline MOX NPs are covered by the coating.

A thickness t12 of the coating 12 preferably is less than 10 nm, and in the present example is 3 nm. A size of individual monocrystalline MOX NPs is referred to by s14. An average monocrystalline MOX NP size is determined by the arithmetic mean of the various monocrystalline MOX NP sizes s14. In the present example, the average monocrystalline MOX NP size is, for example, 13 nm. A fraction of the voids 11 relative to the total volume of the layer preferably is between 30% and 60% (± 10%, e.g., between 30% and 40%). In the present example, the porosity is 35%.

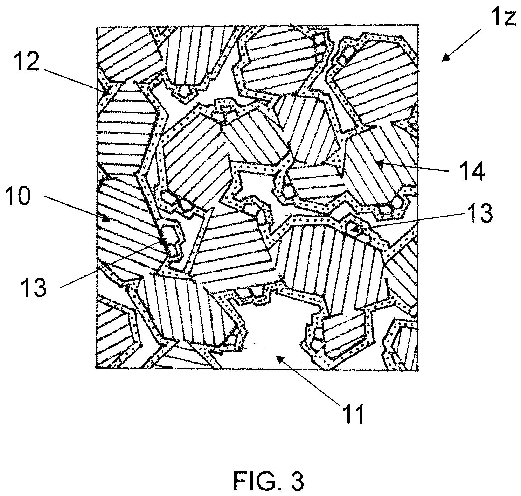

Figure 3 illustrates another zoomed in view of the arbitrary region 1z of Figure 1, again in cross-section. In addition to the material compositions illustrated in the context of Figure 2, a noble metal, such as one of Pt, Pd, Ir, is added to the metal oxide material. Adding in this context may preferably encompasses doping the metal oxide material by the noble metal and / or impregnating the metal oxide material by the noble metal. In the first variant, the noble metal may also appear within MOX NPs, while in the latter variant, the noble metal rather only deposits on the surfaces of the MOX NPs. In the present embodiment of Figure 3, noble metal particles 13 are positioned on the surface of the MOX NPs 14. The noble metal particles 13 serve as nuclei for the material of the coating 12 during the CVD process, and hence act as support for the coating process. The result after coating is illustrated in Figure 3: The coating 12 coats the network of monocrystalline MOC NPs including the noble metal particles 13 on the surfaces of the MOX NPs.

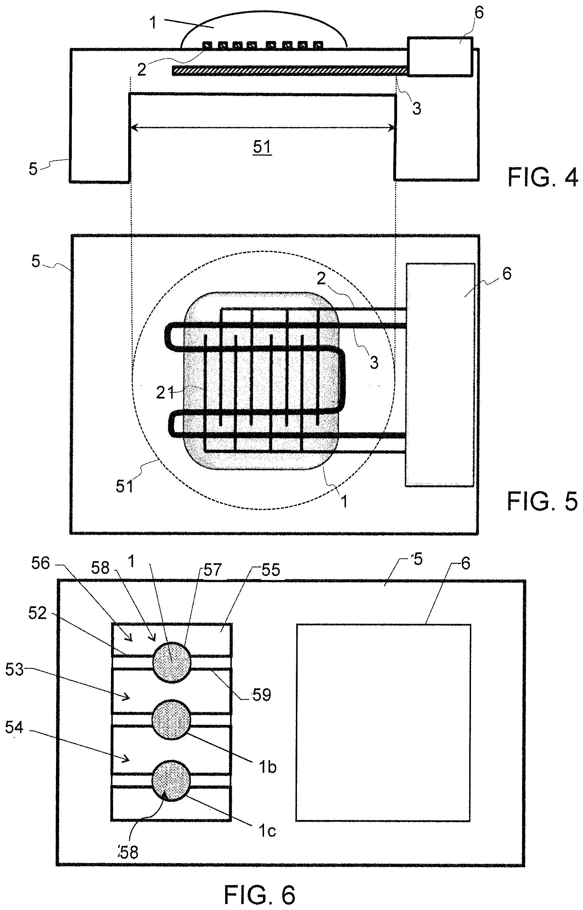

FIG. 4 illustrates a resistive metal oxide gas sensor according to another embodiment of the present invention.

The gas sensor comprises a support structure which presently includes a structured substrate 5, e.g., of silicon. The substrate 5 has a membrane configuration, i.e., the substrate 5 includes a recess 51 for providing a thinned portion of the substrate 5, on which electrodes 2 are arranged. In the thinned portion 51, a heater 3 is provided for heating a porous sensing layer 1 arranged on top of the thinned portion 51 of the substrate 5. The electrodes 2 are arranged on or in the gas sensor, so as to be in electrical communication with a porous sensing layer 1. They may be formed out of a platinum or gold layer, which metals are well suited for forming stable electrodes. The electrodes 2 may for instance be in an interdigitated configuration containing interdigitated fingers 21. Thus, the porous sensing layer 1 may advantageously have shape that spans a region that covers or includes the interdigitated fingers 21 of the electrodes 2 to ensure proper electrical communication therewith.

The heater 3 is in thermal communication with the porous sensing layer 1, to operate the metal oxide material at a target temperature. The heater 3 is a resistive heating element. For example, one may use a heater 3 of tungsten, i.e., a heater 3 comprising at least 50% (± 1%), in particular at least 90% (± 1%), of tungsten, to best withstand high temperatures. Several heaters may be provided, to heat the membrane, or a bridge, see Figure 6, on which the porous sensing layer 1 is arranged. In variants, the heater 3 may be embodied as a hotplate, which is resistively heated, without additional resistive elements being needed. The heater 3 can be used to heat the porous sensing layer 1 and, if necessary, to furthermore control the temperature of the porous sensing layer 1. Thus, no additional temperature sensors need necessarily be provided.

The porous sensing layer 1 extends on an exposed surface of the thinned portion 51 of the substrate 2, i.e., the membrane, thereby covering the electrodes 2 to a large part. The porous sensing layer 2 may for instance have a disk, ovoid or, still, a rectangular shape. All other properties of the porous sensing layer 1 can be taken from the description of Figures 1 to 3.

The gas sensor preferably includes circuitry (integrated therewith), which is electrically connected to the heater 3 and to the electrodes 2. The circuitry preferably forms a controller 6 to heat the heater 3 and perform resistive measurements, i.e., to measure an electrical conductivity and/or resistivity of the porous sensing layer 1 by means of the electrodes 2 that are electrically connected to the controller 6.

In FIG. 6, a gas sensor according to another embodiment is shown in top view, i.e., a multi-pixel gas.

A recess or opening 55 is arranged in the substrate 5, preferably silicon substrate. A bridge structure 56 extends over the recess or opening 55 and is anchored in the substrate 5. The bridge structure 56 includes individual bridges 52, 53, 54 spanning this opening or recess 55. Each bridge 52, 53, 54 comprises a central region 57 forming a hotplate 58 and two arms 59 extending between the central region 57 and the substrate 5, thereby suspending the hotplate 58 over the recess or opening 55.

Each of the bridge structures 56 forms a thin structure with a hotplate. By spanning the recess or opening 55 by means of a bridge and not a continuous thin film cover, the thermal conductance between the hotplate and the substrate 5 is reduced, thus allowing high temperatures to be achieved at low operating power. Further, the thermal mass is reduced, which allows to vary the temperature of the hotplate quickly.

The gas sensor comprises a set of sensing layers 1, 1a and 1b electrically separated from each other. Each sensing layer 1, 1a, 1b is arranged on a dedicated hotplate 58. Each of the sensing layers 1, 1a and 1b comprises a MOX material. The MOX material changes at least one electrical property as a function of the composition of the gas reaching it. The change of the property can be measured in order to obtain information on said composition.

Preferably, at least one of the sensing layers, e.g., the sensing layer 1, is the porous sensing layer 1 as introduced in the previous embodiments, while the other sensing layers 1a, 1b may, for instance, also show different properties as to the presence, the absence, the property of a coating, the porosity, a thickness, the material etc. Thus, several configurations can be contemplated, which enable selectivity of the sensed molecules.

The MOX material of each of the sensing layers 1, 1a, 1b is in electrical communication with a subset of electrodes, which are not visible in FIG. 6. Furthermore, one or more heaters (not visible in FIG. 6) are in thermal communication with the porous sensing layers 1, 1a, 1b. Furthermore, the substrate 5 carries an integrated CMOS circuitry contributing to a controller 6, e.g., including circuitry for driving heaters and processing signals from the electrodes and temperature sensors if any. Advantageously, the processing circuitry 6 is integrated in CMOS technology since the whole device described in this section preferably is compatible with current CMOS manufacturing processes.

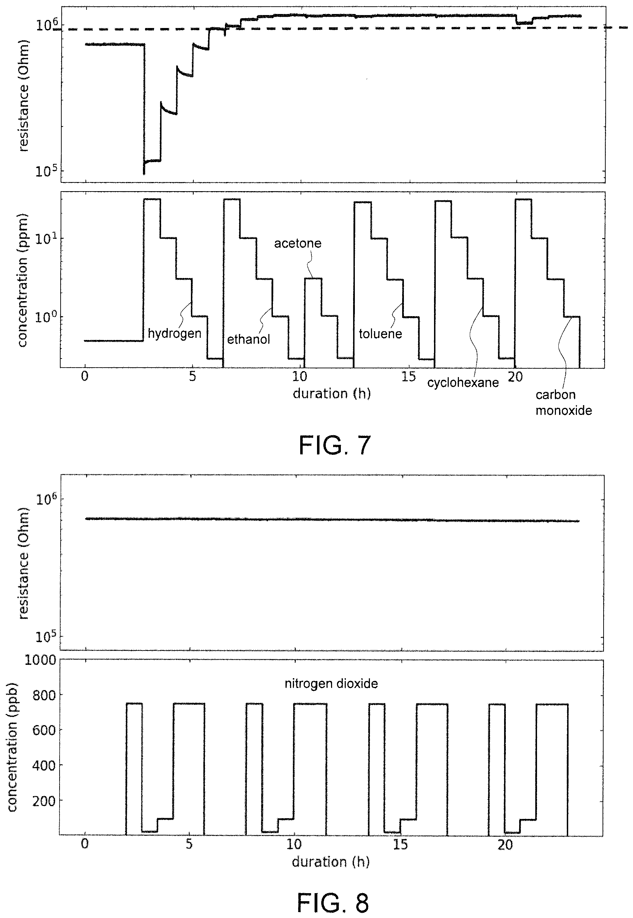

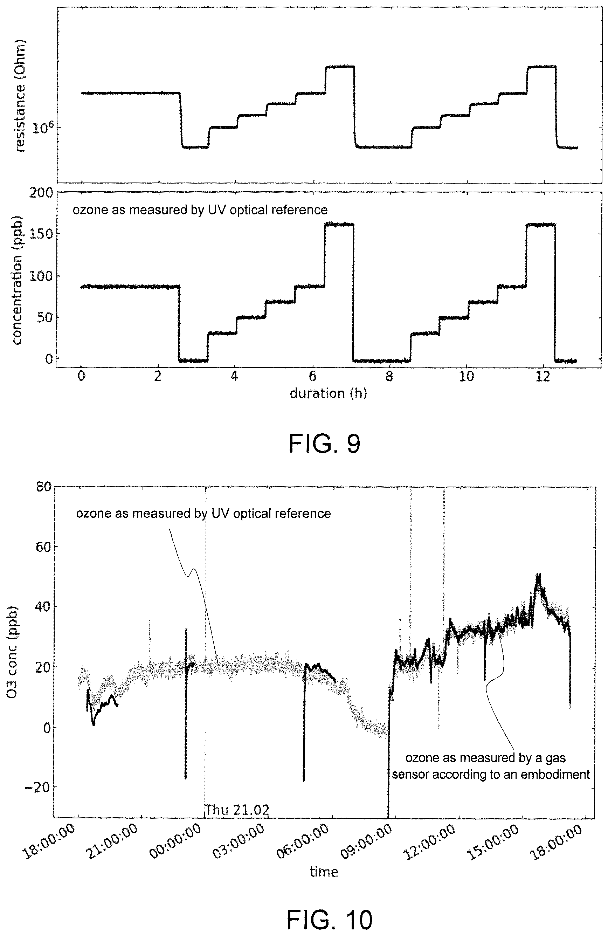

The present approach makes it possible to clearly distinguish concentrations of the target gas/es from other, non-target gases to which the gas sensor according to the present invention may be exposed. For the following plots in Figures 7 to 10, the very same gas sensor was used in accordance with a given embodiment of the present invention, with SnO2 as metal oxide material, a Pt surface impregnation of 3% by weight%, a thickness of the porous sensing layer of 1.5 µm, an average MOX NP size of 13 nm, a SiO2 coating, and a target temperature of 500 °C. FIG. 7 proves the sensitivity to the target gas hydrogen and the absence of cross-sensitivity to typical reducing gases, FIG. 8 proves the absence of cross-sensitivity to a typical oxidizing gas, FIG. 9 proves the sensitivity to the target gas ozone, and FIG. 10 proves the long-term stability.

In detail, FIG. 7 shows, in the upper chart, the resistances of a porous sensing layer of a gas sensor according to said given embodiment, after a sequential exposure to concentrations of various gas molecules over time in a gas mixing system, see lower chart. The gas molecules the gas sensor was exposed to are in the order as depicted in the chart: hydrogen, ethanol, acetone, toluene, cyclohexane and carbon monoxide in a carrier gas of humidified zero-air. As can be derived from the upper chart showing the resistance of the corresponding porous gas sensing layer when exposed to the various gases, only the exposure to hydrogen shows a significant response in the sensing signal taken by the electrodes, i.e., an increase in the electrical conductivity. The exposure of the same gas sensor to the other gases does not lead to any significant change in the sensing signal.

In terms of quantitatively describing the effect, the selectivity of hydrogen versus the other gases is more than 100:1, meaning that when exposed to a given concentration, hydrogen effects a bigger change in the resistance of the porous sensing layer represented by the sensing signal than when exposed to a hundredfold increased concentration of any of the other gases listed. For instance, the dashed line shows that 0.3ppm of hydrogen effect a larger signal (more resistance drop) than 30ppm of any of the other gases. A cross-sensitivity to the other reducing gases is thus designed to be very low.

In the upper chart of FIG. 8 resistances of a porous sensing layer of the same sample as in FIG. 7 are shown, after an exposure to various concentrations of nitrogen dioxide over time in a carrier gas of humidified zero-air and 0.5 ppm hydrogen, see lower chart. The hydrogen is meant to reflect ambient atmospheric hydrogen concentration. As can be derived from the upper chart, the present gas sensor is also not sensitive to nitrogen dioxide given that an increased concentration of nitrogen dioxide does not lead to a change in the resistance of the porous sensing layer.

Presently, the resistance measured in the upper chart of roughly 700 kOhm rather stems from the continuous background concentration of H2 in the gas mixing system, i.e., 0.5 ppm (see FIG. 7 for the same resistance level at 0.5 ppm H2).

The upper chart of FIG. 9 shows the resistances of the same sample as in FIG. 7 and FIG. 8, after an exposure to various concentrations of ozone over time in a carrier gas of humidified zero-air and 0.5 ppm hydrogen, see lower chart. The ozone concentrations of the lower chart were measured by a UV-optical reference device. As can be derived from the upper chart, the present gas sensor is sensitive to ozone and shows fast reaction times. In addition, it can be derived that the present gas sensor has a limit of detection of much less than 20 ppb ozone in view of the stepwise increase of ozone in the atmosphere around the gas sensor of around 20 ppb, which perfectly are reflected in the change of resistance of the porous sensing layer. Note that the change in resistance is positive for oxidizing gases such as ozone. Note also that 20 ppb of ozone effect a much larger change in resistance than 750 ppb of nitrogen dioxide, which again suggests a selectivity of better than 100:1 in favour of ozone vs nitrogen dioxide.

FIG. 10 shows a plot representing an outdoor field measurement of ozone measured by a calibrated gas sensor according to that same given embodiment as used for FIGs. 7 to 9. The time period shown corresponds to a period of 24 hours, taken six months after the gas sensor was calibrated in a gas mixing system with humidified zero air and 0.5 ppm hydrogen and ozone steps such as shown in FIG. 9. In the six months after calibration and, hence, prior to the shown measurement, the gas sensor was randomly switched on and off with a duty cycle of roughly 50%. The grey signal in the 24-hour measurement represents a continuous monitoring by a UV-optical reference device of the actual ozone concentration, while the dark line represents the ozone concentration measured by the gas sensor, after randomly switching on the gas sensor four times for a random duration. Every time after switching on the gas sensor, the sensing signal quickly supplies the correct ozone concentration present at that point in time and follows any subsequent variation of the concentration of ozone. Hence, the gas sensor is proven as long term stable.

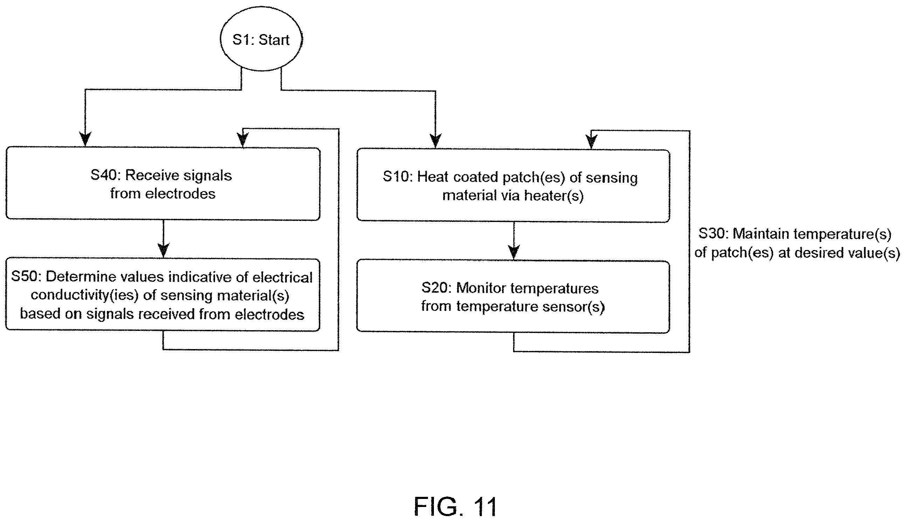

According to another aspect, the invention can further be embodied as a method of operating a resistive MOX gas sensor. Main aspects are briefly discussed in reference to FIG. 11. Basically, in this method, a porous sensing layer 1 is heated to an operating temperature, step S10, FIG. 11. Meanwhile, values indicative of an electrical conductivity of the porous sensing layer are determined (step S50), e.g., thanks to an evaluation unit), and based on signals received S40 from the electrodes. Steps S40, S50 may be continuously (i.e., repeatedly) performed, while heating the coated patch.

As discussed earlier, the porous sensing layer is preferably heated to a temperature that is between 400 °C and 600 °C. In particular, one may, while heating the coated patch, want to maintain a temperature of the coated patch at a desired value. This can notably be achieved thanks to a feedback loop mechanism. This, of course, does not preclude the possibility to occasionally heat the sensor for shorter periods to the target temperature, i.e., intermittent heating.

The above embodiments have been succinctly described in reference to the accompanying drawings and may accommodate a number of variants. Several combinations of the above features may be contemplated.

## Claims

1. A resistive metal oxide gas sensor, comprising
a support structure;
a porous sensing layer (1) arranged on the support structure or partly housed therein;
electrodes (2) in electrical communication with the porous sensing layer (1);
a heater (3) in thermal communication with the porous sensing layer (1), the heater configured to heat the porous sensing layer (1) to a target temperature so as to allow a determination of the presence or the concentration of a target gas based on a sensing signal supplied via the electrodes (2);
wherein:
the porous sensing layer (1) comprises
- a network of interconnected monocrystalline metal oxide nanoparticles (14) ; and
- a gas-selective coating (12) of the network;
the coating (12) comprises one or more of silicon oxide and silicon nitride;
a thickness (t1) of the porous sensing layer (1) is at most 10 µm;
a thickness (t12) of the coating (12) is at most 10 nm; and
the target gas is ozone.

2. The resistive metal oxide gas sensor according to claim 1,
wherein the porous sensing layer (1) is configured to show a void (11) fraction in a range between 30% and 60% denoting a porosity of the porous sensing layer (1).

3. The resistive metal oxide gas sensor according to claim 1 or 2, wherein
the thickness (t12) of the coating (12) is less than 5 nm, and
preferably, the thickness (t12) of the coating (12) is between 1 nm and 3 nm.

4. The resistive metal oxide gas sensor according to any of the preceding claims, wherein
an average size of the monocrystalline metal oxide nanoparticles is at most 100 nm, said average size being preferably less than 50 nm, more preferably between 5 nm and 19 nm, and even more preferably between 10 nm and 15 nm.

5. The resistive metal oxide gas sensor according to any of the preceding claims,
wherein the thickness (t1) of the porous sensing layer (1) is at most 5 µm;
preferably wherein the thickness (t1) of the porous sensing layer (1) is at most 1 µm.

6. The resistive metal oxide gas sensor according to any of the preceding claims, wherein:
the sensor further comprises a controller (6) configured to operate the heater (3) for it to heat the porous sensing layer (1) to said target temperature, the latter being in a temperature range between 400 °C and 600 °C, and preferably between 450 °C and 550 °C;
the sensor preferably comprises a temperature sensor (4) integrated in the sensor and configured to provide a temperature feedback to the controller; and
the controller is preferably integrated in the sensor.

7. The resistive metal oxide gas sensor according to any of the preceding claims,
wherein the metal oxide material comprises one or more of SnO2, In2O3, WO3, TiO2, ZnO, Ga2O3, and Fe203;
preferably wherein the metal oxide material consists of one of SnO2, In2O3, WO3, Ti02, ZnO, Ga203, and Fe2O3.

8. The resistive metal oxide gas sensor according to any of the preceding claims,
wherein the monocrystalline metal oxide nanoparticles (14) comprise a noble metal (13) doping of at most 10 wt%, preferably between 0.3 wt% and 4.0 wt%,
preferably wherein the noble metal (13) is one of Pt, Pd, and Ir.

9. The resistive metal oxide gas sensor according to any of the preceding claims,
wherein the electrodes (2) are arranged on or in the support structure and are at least partly covered by the porous sensing layer (1).

10. The resistive metal oxide gas sensor according to any of the preceding claims,
wherein the coating (12) covers all surfaces of the network accessible by gaseous precursors of the coating material,
preferably wherein the coating (12) covers all surfaces of the network except for surfaces in direct contact with the support structure, the electrodes (2) and the heater (3), if so, and except for surfaces facing voids (13) in the porous sensing layer (1) inaccessible to the gaseous precursors,
preferably wherein the coating (12) covers top surfaces of the network facing the environment prior to coating, and in addition covers buried surfaces within the porous sensing layer (1),
preferably wherein the coating (12) is uniform in its thickness (t12) including a tolerance of ± 50%, and preferably including a tolerance of ± 20%.

11. The resistive metal oxide gas sensor according to any of the preceding claims,
wherein the target gas additionally includes hydrogen.

12. A method for manufacturing a resistive metal oxide gas sensor sensitive to ozone, the method comprising the following sequence of steps:
manufacturing a support structure including electrodes (2) and a heater (3);
depositing monocrystalline metal oxide nanoparticles (14) on the support structure;
annealing or sintering the deposited monocrystalline metal oxide nanoparticles (14), thereby forming a network of interconnected monocrystalline metal oxide nanoparticles (14),
applying a gas-selective coating (12) of a thickness (t12) of at most 10 nm onto the network of interconnected monocrystalline metal oxide nanoparticles (14), the coating (12) comprising one or more of silicon oxide and silicon nitride;
the coated network of interconnected monocrystalline metal oxide nanoparticles (14) contributing to a porous sensing layer (1) of a thickness (t1) of at most 10 µm, which porous sensing layer (1) is arranged on the support structure or partly housed therein such that the electrodes (2) are in electrical communication with the porous sensing layer (1) and such that the heater (3) is in thermal communication with the porous sensing layer (1).

13. The method of claim 12, comprising
providing noble metal particles (13) and doping the monocrystalline metal oxide nanoparticles (14) with the noble metal particles (13), the noble metal particles (13) serving as nuclei for growing the coating (12) on the network of interconnected monocrystalline metal oxide nanoparticles (14).

14. The method of claim 12 or claim 13, comprising
applying the coating (12) by means of a CVD process at a coating temperature in a coating temperature range between 400 °C and 700 °C, and preferably in a coating temperature range between 500 °C and 600 °C.

15. The method of claim 14, comprising generating the coating temperature by operating the heater (3).

16. The method of claim 14 or claim 15,
applying the coating (12) by means of the CVD process by using a gaseous oxygen and/or nitrogen species and a gaseous silicon species as gaseous precursors,
preferably wherein the CVD process is applied at ambient pressure, in particular at a pressure of 1 atm,
preferably wherein the gaseous oxygen species comprises one or more of 02 and 03 and/or the gaseous nitrogen species comprises ammonia, and
preferably wherein the gaseous silicon species is one or more of: silane, dichlorosilane, tetraethylorthosilicate, or TEOS, hexamethyldisiloxane, or HMDSO, hexamethylcyclotrisiloxan, or D3, octamethylcyclotetrasiloxan, or D4, and decamethylcyclopentasiloxane, or D5.

17. A method for operating a resistive metal oxide gas sensor according to any one of the preceding claims 1 to 11, or as obtained in accordance with the method of any of the preceding claims 12 to 16, the method comprising:
taking a measurement including:
operating the heater (3) to heat the porous sensing layer (1) to the target temperature; and
determining a presence or a concentration of the target gas based on the sensing signal received from the electrodes (2), preferably while heating the porous sensing layer (1).

18. The method of claim 17, further comprising, prior to taking the measurement:
placing the gas sensor in a gas mixture including hydrogen and calibrating the gas sensor by taking into account a hydrogen background concentration as expected in the measurement environment.
